(19)
Europäisches Patentamt
European Patent Office
Office européen des brevets

(11) Numéro de publication : 0 399 858 B1

(12) # FASCICULE DE BREVET EUROPEEN

(45) Date de publication du fascicule du brevet : **08.07.92 Bulletin 92/28**

(51) Int. Cl.$^5$ : **A61K 31/135,** A61K 47/20

(21) Numéro de dépôt : **90401058.4**

(22) Date de dépôt : **19.04.90**

(54) **Composition nettoyante, en particulier à base de naftifine et/ou de terbinafine.**

(30) Priorité : **03.05.89 FR 8905909**

(43) Date de publication de la demande : **28.11.90 Bulletin 90/48**

(45) Mention de la délivrance du brevet : **08.07.92 Bulletin 92/28**

(84) Etats contractants désignés : **AT BE CH DE ES FR GB IT LI NL SE**

(56) Documents cités : **FR-A- 2 572 933**

(73) Titulaire : **L'OREAL**
**14, Rue Royale**
**F-75008 Paris (FR)**

(72) Inventeur : **Laugier, Jean-Pierre**
**22, rue des Gouttières**
**F-92160 Antony (FR)**
Inventeur : **Fanchon, Chantal**
**105, Quai Branly**
**F-75015 Paris (FR)**
Inventeur : **Jomard, André**
**114, Chemin des Plantiers**
**F-06370 Mouans-Sartoux (FR)**
Inventeur : **Shroot, Braham**
**Villa 35, Hameau de Val Bosquet**
**Chemin de Val Bosquet, F-06600 Antibes (FR)**
Inventeur : **Ringenbach, François**
**60 Bis, boulevard Maréchal Joffre**
**F-92340 Bourg La Reine (FR)**

(74) Mandataire : **Peuscet, Jacques et al**
**Cabinet Peuscet 68, rue d'Hauteville**
**F-75010 Paris (FR)**

EP 0 399 858 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles compositions nettoyantes comportant, en solution aqueuse exempte d'huile, au moins un composant antifongique et au moins un agent tensio-actif.

Il est déjà connu d'utiliser par voie topique des compositions comportant un composant antifongique auquel est associé un agent tensio-actif. La demande de brevet EP-A-156507 décrit des solutions comportant des produits antifongiques comme les dérivés d'imidazole, en particulier le miconazole et le nitrate de miconazole. Ces composés sont quasiment insolubles dans l'eau et nécessitent de ce fait l'apport de solvants alcooliques en quantité importante. Il en résulte que ces solutions ne présentent pas de propriétés moussantes et détergentes suffisantes.

La demande de brevet JP-A-56.025.108 fait état, quant à elle, de solutions dermatologiques comportant du bis-2-pyridinethiol-1-oxyde de zinc ainsi que des agents tensio-actifs ayant fonction d'augmenter la solubilité et la pénétration cutanée de cet antifongique. Cependant, cette solubilisation provoque en même temps une augmentation des propriétés irritantes de l'anion pyridinethiol-1-oxyde.

Il est, de plus, connu d'utiliser des composants antifongiques sous forme de crème ou d'onguent dans lesquels lesdits antifongiques sont dispersés ou solubilisés. De telles préparations ont l'avantage de permettre d'obtenir de bons résultats en ce qui concerne les traitements cutanés, ainsi que de permettre d'utiliser certains constituants dermatologiques peu solubles dans l'eau, mais solubles dans des phases grasses ; elles présentent, néanmoins, des inconvénients au niveau de leur élimination après application, car elles nécessitent souvent un assez long rincage à l'eau. Il en résulte, parfois, des irritations secondaires dues à un contact trop prolongé des composants antifongiques avec la peau.

Pour pallier cet inconvénient, la demanderesse a proposé, dans sa demande de brevet FR-A-2 515 034, d'incorporer le constituant dermatologiquement actif dans une solution huileuse, qui a de bonnes caractéristiques moussantes et nettoyantes et peut être assez facilement éliminée par simple addition d'eau. Il est proposé notamment, dans ce document, d'utiliser comme constituant antifongique dans une telle solution, de l'éconazole ou du miconazole, constituants qui sont tous deux solubles dans l'huile et insolubles dans l'eau.

La présente invention a pour but de proposer une nouvelle amélioration des compositions nettoyantes antifongiques à usage pharmaceutique ou vétérinaire ; à cet effet, on propose des compositions nettoyantes comportant des composants antifongiques hydrosolubles, ce qui permet de les utiliser en phase aqueuse et d'obtenir leur élimination très rapide par rinçage après application ; on évite ainsi les causes d'irritations secondaires tout en bénéficiant de bonnes propriétés antifongiques. L'invention propose essentiellement d'utiliser, comme composants antifongiques hydrosolubles, des composants choisis dans la famille des allylamines, en les associant avec des agents tensio-actifs hydrosolubles anioniques.

De façon surprenante, il a été constaté par la demanderesse, que de telles compositions nettoyantes, utilisées par nature dans un mode de traitement topique de courte durée, permettent d'obtenir non seulement une activité antifongique, après rinçage, identique à celle d'une crème non rincée et de même concentration en principe actif, mais aussi une évolution clinique de la lésion mycotique meilleure que celle obtenue avec une crème non rincée de même concentration. De plus, il s'avère que ces compositions ont de bonnes propriétés moussantes.

L'invention a donc pour objet une composition nettoyante, à usage pharmaceutique ou vétérinaire pour le traitement des dermatophytoses par voie topique, comportant, en solution aqueuse, au moins un composant antifongique et au moins un agent tensio-actif, cette composition nettoyante étant constituée d'une solution contenant, comme antifongique, au moins une allylamine et, comme agent tensio-actif, au moins un tensio-actif anionique.

De façon avantageuse, cette composition contient, comme allylamine(s), la naftifine de formule

et/ou la terbinafine de formule

et/ou leurs sels (notamment leur chlorhydrate).

La composition selon l'invention contient avantageusement de Ø,Ø1 à 2,5 % en poids d'allylamine(s) par rapport au poids total de la composition.

La composition comporte préférentiellement de 2 à 15 % en poids d'agent(s) tensio-actif(s) anionique(s) par rapport au poids total de la composition.

Parmi les agents tensio-actifs anioniques utilisables, on peut citer, par exemple, les alkylsulfates, les alkylsulfonates, les alkyléther-sulfates, les alkylsulfosuccinates, les alkyléther-sulfosuccinates, les alkyléther-phosphates, les alkylsarcosinates, les alkylpolypeptidates, les alkylcarboxylates et les sels de polyaminoacidés.

De préférence, le (ou les) agent(s) tensio-actif(s) anionique(s) de la composition selon l'invention est (ou sont) choisi(s) dans le groupe formé par les alkylsulfates et les alkyléther-sulfates définis par la formule générale :

$$CH_3-(CH_2)_n-(O-CH_2-CH_2)_x-O-SO_3^- X^+$$

formule dans laquelle n a une valeur comprise entre 9 et 23, x a une valeur comprise entre Ø et 8, et X est un ion métallique monovalent ou un ammonium, substitué ou non. Préférentiellement, n a une valeur comprise entre 11 et 15 ; x a une valeur comprise entre Ø et 3; X est le sodium, le potassium ou l'ammonium.

De façon avantageuse, la composition selon l'invention peut contenir au moins un agent tensio-actif non-ionique pris dans le groupe formé par les dérivés polyoxyéthylénés du sorbitan, les alkylpolyglycol-éthers et les alkylpolyglycérol-éthers.

La composition peut aussi contenir au moins un additif pris dans le groupe formé par les agents stabilisateurs de mousse non-ioniques, les antioxydants, les épaississants, les conservateurs, les agents chélatants, et les polymères cationiques. L'agent stabilisateur de mousse non-ionique peut être un amide d'acides gras de coprah ; le conservateur peut être l'alcool benzylique.

Les exemples suivants sont destinés à illustrer l'invention sans pour autant la limiter.

EXEMPLE 1 :

Pour le traitement du pityriasis capitis, on prépare la lotion suivante :

- Lauryléther sulfate de sodium oxyéthyléné à 2,2 moles d'oxyde d'éthylène (30 % de matière active en solution aqueuse) ...... 15,00 g
- Laurylsulfate de triéthanolamine vendu par la société "HENKEL"® sous la dénomination "Texapon T42" (40 % de matière active) ........ 18,00 g
- Chlorhydrate de terbinafine ..... 1,00 g
- Alcool benzylique .............. 1,00 g
- Eau purifiée q.s.p. ............. 100,00 g

On applique une dose de 5 ml de cette lotion moussante sur des cheveux mouillés, puis on laisse agir cette lotion pendant 2 minutes. Après avoir ajouté un peu d'eau tiède et fait mousser la lotion, on l'élimine par rinçage. On renouvelle ensuite une deuxième fois cette application et on rince abondamment.

Il est préconisé pour réaliser un traitement efficace, d'effectuer deux applications par semaine, pendant deux semaines. Pour un simple traitement d'entretien, une application par semaine suffit le plus souvent.

Il a été constaté que l'utilisation de cette lotion permettait à la fois de faire disparaître les plaques squameuses sur les lésions et d'espacer considérablement les rechutes. De plus, du fait de sa rinçabilité, cette lotion n'a aucun effet secondaire notable.

Les propriétés de cette composition moussante ont été, on outre, évaluées à partir d'une étude étalée sur vingt et un jours et portant sur des cobayes présentant une mycose cutanée induite, au premier jour de l'étude, par inoculation de Trichophyton mentagrophytes. Les cobayes ont été traités avec la composition, à partir du cinquième jour après l'inoculation, le traitement ayant lieu une fois par jour pendant deux périodes ce cinq jours séparées par une période de deux jours.

Deux paramètres ont servi à évaluer l'activité de la composition, le premier paramètre étant obtenu à partir d'un examen clinique de la lésion mycotique, le deuxième paramètre caractérisant la façon dont la composition ralentit, dans un milieu de culture pour dermatophytes, des cultures faites à partir de poils prélevés sur les cobayes.

L'examen clinique de la lésion mycotique comprenait des observations relatives à la présence d'érythème, d'oedème, de desquamation, d'alopécie, de croutes, de prurit, et d'exsudation. Ces observations, effectuées régulièrement entre le cinquième et le vingt et unième jour de l'étude, sont évaluées et notées selon une notation allant de 0 à 4 selon la gravité du signe clinique pour les cinq premiers signes cliniques ; le prurit et l'exsudation sont, quant à eux, notés 0 ou 1 selon leur absence ou leur présence. Pour chaque examen, un score clinique global est déterminé en sommant les scores des différents signes cliniques, l'ensemble des scores cliniques ainsi obtenus permettant de déterminer statistiquement, par analyse de variance et test de Student-Newman-Keuls, un score clinique global représentatif.

Dans le même temps, on évalue un score global d'inhibition en notant l'importance de la croissance de cultures ensemensées par des poils prelevés tout au long des 21 jours de l'étude sur des cobayes traités. Après ensemencement, le milieu de culture (gélose/ chloramphénicol/actidione) est mis en incubation pendant 7 jours à 26°C. A la fin de cette période, la croissance est évaluée : une croissance très importante est notée par un score d'inhibition de 0 % ; une croissance nulle est notée par un score d'inhibition de 100 %. Un score global représentatif du pouvoir d'inhibition est ensuite déterminé statistiquement par analyse de variance et test de Student-Newman-Keuls.

Parallèlement et suivant le même protocole d'étude, on a testé une composition sous forme de crème non rincée comportant 1 % de terbinafine. La crème correspond à la formulation suivante :

- Stéarate de polyethylène glycol 100 ............................ 3,00 %
- Stéarate de polyéthylène glycol 50 ............................ 3,00 %
- Alcool stéarylique ............. 3,00 %
- Alcool cétylique ............... 3,00 %
- Perhydrosqualène ............... 12,00 %
- Triéthanolamine ................ 0,80 %
- Glycérine ...................... 3,00 %
- Hydroxyéthylcellulose .......... 0,20 %
- Chlorhydrate de terbinafine ..... 1,00 %
- Eau purifiée q.s.p. ............ 100,00 %

où les pourcentages sont donnés en poids par rapport au poids total de la composition.

Les résultats obtenus sont donnés dans le tableau suivant :

| | Composition à 1 % de terbinafine de l'exemple 1 | Composition à 1 % de terbinafine sous forme de crème non rincée |
|---|---|---|
| Pouvoir d'inhibition des cultures | 103,7 | 108,4 |
| Score clinique global | 11,67 | 26,4 |

Il ressort de ce tableau, que le score clinique obtenu avec la composition selon l'invention, bien que rincée après application, est bien meilleur que celui obtenu avec une crème non rincée, les pouvoirs d'inhibition étant sensiblement équivalents.

EXEMPLE 2 :

On prépare un gel de douche pour le traitement du pityriasis versicolor (épidermomycose chronique et récidivante fréquente) ayant la formulation suivante :

- Lauryléther sulfate de sodium (70 % de matière active en solution aqueuse) vendu sous la dénomination "Texapon® N70" par la société "HENKEL" ........ 15,00 g
- Diéthanolamides d'acides gras de coprah vendus sous la dénomination "Comperlan® KD" par la société "HENKEL" ................ 3,00 g
- Glycérides caprique et caprylique vendus sous la dénomination "Softigen® 767" par la Société "Dynamit Nobel" ................. 4,00 g
- Chlorure de sodium .............. 2,00 g
- Chlorhydrate de terbinafine ..... 0,50 g
- Eau purifiée q.s.p. ............. 100,00 g.

Cette composition s'applique en laissant agir, pendant environ 2 à 5 minutes, sur les zones atteintes, deux noix de gel étalées sur les lésions à l'aide d'un gant de toilette humide. On émulsionne ensuite à l'eau tiède, puis l'on rince abondamment.

Il est conseillé de répéter le traitement pendant deux semaines, deux à trois fois par semaine, selon la gravité des lésions. Pour les cas rebelles, il est possible d'effectuer une application quotidienne pendant une semaine.

Cette lotion est particulièrement bien adaptée au traitement des levures localisées sur le tronc d'un individu. Il est à noter que cette lotion ne présente pas les désagréments des lotions au sulfure de sélénium, qui ont une mauvaise odeur.

Après une semaine de traitement quotidien, la guérison mycologique peut être mise en évidence sur un sujet atteint par l'absence de fluorescence au cours d' une observation des lésions à la lumière de WOOD.

EXEMPLE 3 :

On prépare la lotion antipelliculaire moussante suivante :

- Laurylsulfate de triéthanolamine (40 % de matière active en solution aqueuse) ........................ 30,00 g
- Sel disodique de l'acide éthylène-diamine-tétraacétique .. 0,1 g
- Mélange d'esters lauriques de sorbitol et d'anhydrides de sorbitol vendu par la société "ICI Americas" sous la dénomination "Tween® 20" .............. 3,00 g
- Chlorydrate de terbinafine ...... 1,20 g
- Eau purifiée q.s.p. ............. 100,00 g

Cette lotion, qui peut être appliquée de la même façon que celle de l'exemple 1, peut aussi être appliquée une fois par jour pendant six jours puis deux fois par semaine comme traitement d'entretien. Après une semaine de traitement quotidien, on peut noter une amélioration nette de l'état pelliculaire.

Cette lotion est particulièrement adaptée, chez le cheval, au traitement des teignes dues au trichophyton équinum, en complément d'un traitement systémique à la terbinafine.

EXEMPLE 4 :

Pour le traitement des mycoses des plis, on prépare la lotion à fort pouvoir moussant suivante :

- Octoxynol-2 ethane sulfonate de sodium (28 % de matière active en solution aqueuse) vendu par la société "ROHM & HAAS" sous la dénomination "Triton® X-200" ...... 10,00 g
- Laurylsarcosinate de sodium (30 % de matière active en solution aqueuse) vendu sous la dénomination "ORAMIX® L30" par la société "SEPPIC" .......... 12,50 g
- Glycérol ........................ 2,50 g
- Sel disodique de l'acide éthylène diamine-tétraacétique ............ 0,10 g
- Diéthanolamides d'acides gras de coprah vendu par la société "HENKEL" sous la dénomination "Comperlan® KD" 2,00 g
- Chlorydrate de terbinafine ...... 1,50 g
- Hexylène glycol ................. 0,50 g
- Eau purifiée q.s.p. ............. 100,00 g.

Cette lotion est appliquée, quotidiennement ou deux fois par jour, jusqu'à disparition des lésions, sur des lésions d'intertrigo sous-mammaire, génital ou interdigital ; elle est appliquée sur les lésions pendant deux à trois minutes à raison de Ø,1 gramme par cm2. Après application, la lotion est émulsionnée puis rincée abondamment. Les lésions sont bien séchées après rinçage.

Il est à noter que cette lotion est adaptée aussi bien au traitement des mycoses macérées qu'au traitement des mycoses sèches.

Après 1Ø jours de traitement, on observe une disparition des lésions.

EXEMPLE 5 :

On prépare une lotion moussante, pour le traitement du pityriasis versicolor, ayant la formulation suivante :

- Lauryléther sulfate de sodium (7Ø % de matière active en solution aqueuse) vendu sous la dénomination "Texapon® N7Ø" par la société "HENKEL"................. 15,ØØ g
- Laurylsarcosinate de sodium (3Ø % de matière active en solution aqueuse) vendu sous la dénomination "Oramix® L3Ø" par la société "SEPPIC" ................ 12,ØØ g
- Copolymère de chlorure de diméthyl diallyle ammonium et d'acrylamide connu sous le nom générique de "Polyquaternium 7", nom adopté par l'association C.T.F.A. (Cosmetic Toiletry and Fragance Association), et vendu sous la dénomination "Merquat® 55Ø" par la société "MERCK" ......................... 2,ØØ g
- Chlorure de sodium .............. 2,ØØ g
- Alcool benzylique ................ 1,ØØ g
- Chlorhydrate de naftifine ....... Ø,5Ø g
- Eau purifiée q.s.p........... 100,00 g.

Cette lotion s'applique de la façon qui est décrite dans l'exemple 1.

EXEMPLE 6

On a préparé une lotion pour le traitement de Pityriasis capitis ayant la même composition que celle de l'exemple 1 sauf que l'on a remplacé le chlorhydrate de terbinafine par le chlorhydrate de naftifine. On a ensuite traité les cheveux mouillés de la même façon et effectué le même examen clinique que dans l'exemple 1. On a obtenu les résultats donnés dans le tableau ci-dessous.

| | Lotion à 1 % de naftifine |
|---|---|
| Pouvoir d'inhibition des cultures | 84,2 % |
| Score clinique global | 25,2 |

EXEMPLE 7 (comparatif)

On a également préparé une lotion pour le traitement de Pityriasis capitis ayant la même composition que celle de l'exemple 1 sauf que l'on a remplacé le chlorhydrate de terbinafine par un chlorhydrate d'éconazole, l'éconazole n'étant pas une allylamine. On a également préparé une crème non rincée contenant 1 % d'éconazole au lieu de terbinafine. On a effectué les mêmes traitements et les mêmes examens cliniques que dans l'exemple 1. On a obtenu les résultats donnés dans le tableau ci-dessous.

| | Crème à 1 % d'éconazole non rincée | Lotion à 1 % d'éconazole rincée |
|---|---|---|
| Pouvoir d'inhibition des cultures | 100 % | 31,7 % |
| Score clinique global | 20,2 ± 4,9 | 27,4 ± 4,4 |

Ce tableau montre que lorsque l'on n'utilise pas une allylamine comme produit antifongique, on n'obtient pas la même efficacité avec une lotion rincée qu'avec une crème non rincée.

**Revendications**

1. Composition nettoyante a action pharmaceutique ou vétérinaire, pour le traitement des dermatophytoses par voie topique, comportant en solution aqueuse au moins un composant antifongique et au moins un agent tensio-actif, caractérisée par le fait qu'elle est constituée d'une solution contenant comme antifongique, au moins une allylamine, et, comme agent tensio-actif, au moins un tensio-actif anionique.

2. Composition selon la revendication 1, caractérisée par le fait qu'elle contient, comme allylamine(s), la naftifine de formule

$$CH_3\text{-}N(CH_2\text{-}C_{10}H_7)\text{-}CH_2\text{-}CH{=}CH\text{-}C_6H_5$$

et/ou la terbinafine de formule

$$CH_3\text{-}N(CH_2\text{-}C_{10}H_7)\text{-}CH_2\text{-}CH{=}CH\text{-}C{\equiv}C\text{-}C(CH_3)_3$$

et/ou leurs sels.

3. Composition selon l'une des revendications 1 ou 2, caractérisée par le fait qu'elle contient de 0,01 à 2,5 % en poids d'allylamine(s) par rapport au poids total de la composition.

4. Composition selon l'une des revendications 1 à 3, caractérisée par le fait qu'elle contient de 2 à 15 % en poids d'agent(s) tensioactif(s) anionique(s) par rapport au poids total de la composition.

5. Composition selon l'une des revendications 1 à 4, caractérisée par le fait que le (ou les) agent(s) tensio-actif(s) anionique(s) est (ou sont) choisis dans le groupe formé par les alkylsulfates, les alkylsulfonates, les alkyléther-sulfates, les alkylsulfosuccinates, les alkyléther-sulfosuccinates, les alkyléther-phosphates, les alkylsarcosinates, les alkylpolypeptidates, les alkylcarboxylates et les sels de polyaminoacidés.

6. Composition selon la revendication 5, caractérisée par le fait que le (ou les) agent(s) tensio-actif(s) de la composition selon l'invention est (ou sont) choisi(s) dans le groupe formé par les alkylsulfates et les alkyléther-sulfates définis par la formule générale :

$$CH_3\text{-}(CH_2)_n\text{-}(O\text{-}CH_2\text{-}CH_2)_x\text{-}O\text{-}SO_3^-X^+$$

formule dans laquelle n a une valeur comprise entre 9 et 23, x a une valeur comprise entre Ø et 8, X est un ion métallique monovalent ou un ammonium, substitué ou non.

7. Composition selon la revendication 6, caractérisée par le fait que n est compris entre 11 et 15.

8. Composition selon l'une des revendications 6 ou 7, caractérisée par le fait que x est compris entre Ø et 3.

9. Composition selon l'une des revendications 6 à 8, caractérisée par le fait que X est l'ammonium, le sodium ou le potassium.

10. Composition selon l'une des revendications 1 à 9, caractérisée par le fait qu'elle contient au moins un agent tensio-actif non ionique pris dans le groupe formé par les dérivés polyoxyéthylénés du sorbitan, les alkylpolyglycol-éthers ou les alkylpolyglycérol-éthers.

11. Composition selon l'une des revendications 1 à 1Ø, caractérisée par le fait qu'elle contient au moins un additif pris dans le groupe formé par les agents stabilisateurs de mousse non-ioniques, les antioxydants, les épaississants, les conservateurs, les agents chélatants, et les polymères cationiques.

## Claims

1. Cleansing composition having a pharmaceutical or veterinary action for the treatment of determatophytoses by topical application, comprising, in aqueous solution, at least one antifungal compound and at least one surface-active agent, characterised in that it consists of a solution containing at least one allylamine, as antifungal compound, and at least one anionic surfactant, as surface-active agent.

2. Composition according to Claim 1, characterised in that it contains, as allylamine(s), naftifine of formula

and/or terbinafine of formula

and/or their salts.

3. Composition according to one of Claims 1 or 2, characterised in that it contains from 0.01 to 2.5 % by weight of allylamine(s) relative to the total weight of the composition.

4. Composition according to one of Claims 1 to 3, characterised in that it contains from 2 to 15 % by weight of anionic surface-active agent (s) relative to the total weight of the composition.

5. Composition according to one of Claims 1 to 4, characterised in that the anionic surface-active agent(s) is (or are) chosen from the group formed by alkylsulphate, alkylsulphonates, alkyl ether-sulphates, alkylsulphosuccinates, alkyl ether-sulphosuccinates, alkyl ether-phosphates, alkylsarcosinates, alkylpolypeptidates, alkylcarboxylates and the salts of polyaminoacids.

6. Composition according to Claim 5, characterised in that the surface-active agent(s) in the composition according to the invention is (are) chosen from the group formed by the alkylsulphates and the alkyl ethersulphates defined by the general formula:

$$CH_3-(CH_2)_n-(O-CH_2-CH_2)_x-O-SO_3^-X^+$$

in which formula n has a value between 9 and 23, x has a value between 0 and 8 and x is a monovalent metal ion or a substituted or unsubstituted ammonium.

7. Composition according to Claim 6, characterised in that n is between 11 and 15.

8. Composition according to one of Claims 6 or 7, characterised in that x is between 0 and 3.

9. Composition according to one of Claims 6 to 8, characterised in that x is ammonium, sodium or potassium.

10. Composition according to one of Claims 1 to 9, characterised in that it contains at least one nonionic surface-active agent taken from the group formed by the polyoxyethylenated derivatives of sorbitan, alkyl polyglycol ethers or alkyl polyglycerol ethers.

11. Compositon according to one of Claims 1 to 10, characterised in that it contains at least one additive taken from the group formed by nonionic foam stabilisers, antioxidants, thickeners, preservatives, chelating agents and cationic polymers.

## Patentansprüche

1. Pharmazeutisches oder veterinärmedizinisches Reinigungsmittel zur topischen Behandlung von Dermatophytosen, das in wässriger Lösung mindestens eine Antifungus-Verbindung und mindestens ein oberflächenaktlves Agens umfaßt, dadurch gekennzeichnet, daß es aus einer Lösung besteht, welche als Antifungus-Verbindung mindestens ein Allylamin und als oberflächenaktives Agens mindestens ein anionisches oberflächenaktives Agens enthält.

2. Mittel nach Anspruch 1, dadurch gekennzeichnet, daß es als Allylamin(e) Naftifin der Formel

und/oder Terbinafin der Formel

und/oder die Sälze davon enthält.

3. Mittel nach einem der Ansprüche 1 oder 2, dadurch gekennzeichnet, daß es 0,01 bis 2,5 Gew.-% Allylamin(e) bezogen auf das Gesamtgewicht des Mittels enthält.

4. Mittel nach einem der Ansprüche 1 bis 3, dadurch gekennzeichnet, daß es 2 bis 15 Gew.-% eines oder mehrerer anionischer oberflächenaktiver Agentien bezogen auf das Gesamtgewicht des Mittels enthält.

5. Mittel nach einem der Ansprüche 1 bis 4, dadurch gekennzeichnet, daß das (die) oberflächenaktive(n) anionische(n) Agens (Agentien) ausgewählt ist (sind) unter Alkylsulfaten, Alkylsulfonaten, Alkyläthersulfaten, Alkylsulfosucclnaten, Alkyläthersulfosuccinaten, Alkylätherphosphaten, Alkylsarcosinaten, Alkylpolyptidaten, Alkylcarboxylaten und den Salzen von Polyaminosäuren.

6. Mittel nach Anspruch 5, dadurch gekennzeichnet, daß das (die) oberflächenaktive(n) Agens (Agentien) des erfindungsgemäßen Mittels ausgewählt ist (sind) unter Alkylsulfaten und Alkyläthersulfaten gemäß der all-

gemeinen Formel

$$CH_3-(CH_2)_n-(O-CH_2-CH_2)_x-O-SO_3^-X^+$$

worin n einen Wert von 9 bis 23 einnimmt, x einen Wert von 0 bis 8 einnimmt und X ein monovalentes Metallion oder ein gegebenenfalls substituiertes Ammoniumion bedeutet.

7. Mittel nach Anspruch 6, dadurch gekennzeichnet, daß n für einen Wert von 11 bis 15 steht.

8. Mittel nach einem der Ansprüche 6 oder 7, dadurch gekennzeichnet, daß x für einen Wert von 0 bis 3 steht.

9. Mittel nach einem der Ansprüche 6 bis 8, dadurch gekennzeichnet, daß X für Ammonium, Natrium oder Kalium steht.

10. Mittel nach einem der Ansprüche 1 bis 9, dadurch gekennzeichnet, daß es mindestens ein nichtionisches,oberflächenaktives Agens, ausgewählt unter polyoxyäthylenierten Derivaten von Sorbitan, Alkylpolyglykoläthern oder Alkylpolyglycerinäthern, enthält.

11. Mittel nach einem der Ansprüche 1 bis 10, dadurch gekennzeichnet, daß es mindestens einen Zusatz, ausgewählt unter nichtionischen Schaumstabilisierungsmitteln, Antioxidantien, Verdickungsmitteln, Konservierungsmitteln,chelatbildenden Mitteln und kationischen Polymeren,enthält.